# EUROPEAN PATENT SPECIFICATION

(11) **EP 3 877 013 B1**
(45) Date of publication and mention of the grant of the patent: **16.07.2025**
(21) Application number: 19813414.0
(22) Date of filing: 08.11.2019
(51) Int. Cl.: A61L 27/18, A61L 27/24, A61L 27/48, A61L 27/54, A61L 27/56, B33Y 70/00, B33Y 80/00

(54) **BONE SIALOPROTEIN FUNCTIONALIZED MATERIALS FOR DIRECTED BONE REGENERATION**
FUNKTIONALISIERTE KNOCHENSIALOPROTEINMATERIALIEN FÜR GERICHTETE KNOCHENREGENERATION
MATÉRIAUX FONCTIONNALISÉS À LA SIALOPROTÉINE OSSEUSE POUR LA RÉGÉNÉRATION OSSEUSE DIRIGÉE

(30) Priority: 08.11.2018 DE 102018128017; 09.09.2019 DE 102019124159
(43) Date of publication of application: 15.09.2021
(73) Proprietor: Immundiagnostik AG, 64625 Bensheim (DE); Johannes Gutenberg-Universität Mainz, 55122 Mainz (DE)
(72) Inventor: ARMBRUSTER, Franz Paul, 64624 Bensheim (DE); ROMMENS, Pol Maria, 55128 Mainz (DE); RITZ, Ulrike, 55218 Ingelheim (DE)
(74) Representative: Benedum, Ulrich Max
(86) International application number: PCT/EP2019/080768
(87) International publication number: WO 2020/094880

(56) References cited:
- EP-A1- 3 231 453
- EP-A2- 2 269 663
- SOPHIA P. PILIPCHUK ET AL: "Integration of 3D Printed and Micropatterned Polycaprolactone Scaffolds for Guidance of Oriented Collagenous Tissue Formation In Vivo", ADVANCED HEALTHCARE MATERIALS, vol. 5, no. 6, 1 March 2016 (2016-03-01), DE, pages 676 - 687, XP055669767, ISSN: 2192-2640, DOI: 10.1002/adhm.201500758
- WAILEN D. CHAN ET AL: "Modification of polymer networks with bone sialoprotein promotes cell attachment and spreading", JOURNAL OF BIOMEDICAL MATERIALS RESEARCH PART A, vol. 9999A, 1 January 2010 (2010-01-01), pages NA - NA, XP055669759, ISSN: 1549-3296, DOI: 10.1002/jbm.a.32715

## Description

### FIELD OF THE INVENTION

The invention relates to 3D-printed composites comprising a resorbable polylactide (PLA) body, and in particular to compositions for accelerating and improving the healing of bone and soft tissue lesions.

### BACKGROUND OF THE INVENTION

The stability of bone implants depends greatly on their ingrowth properties. For example, for dental implants to work, there must be sufficient bone in the jaw, and the bone has to be strong enough to hold and support the implant. Where there is insufficient or inadequate maxillary or mandibular bone in terms of depth or thickness, grafts are used in prosthetic dentistry to provide secure integration with the dental implant Conventional grafts include the patient's own bone (autografts), processed bone from cadaver (allografts), bovine bone or coral (xenografts), and synthetic bone-like or bone-mimetic materials.

The bone morphogenic proteins (BMP) are the only growth factors known to induce bone formation heterotopically. Supplementary doses of BMP boost the bone healing by inducing undifferentiated mesenchymal cells to differentiate into osteoblasts. Bone grafts and implants however shall result in a livevascular bone which is very much like natural US-A 5 478 237 (Ishikawa) discloses an implant coated with a layer of hydroxyapatite, WO 021078759 (Stratec Medical AG) an implant having a layer of a porous metal oxide comprising amorphous and nanocrystallme calcium phosphate and hydroxyapatite, WO 02/085250 (KERWMED GrnbH) an implant wherein a coating of resorbable calcium phosphate phases contains adhesion and signal proteins such as bone siaioprotein (BSP), bone morphogenic protein (BMP), fibronectin, osteopontin (OPN), ICAM-I, VCAM and derivatives thereof. Further grafts and implants of this type are described in EP 1 166 804 A2 (Merck, Darmstadt) and WO 99/08730 (Children's Medical Center Corporation) DE 100 37 850 A1 (Jenissen H) and WO 03/059407 A1 (Straumann Holding AG) describe grafts and Implants treated with ubiqultin or transforming growth factor (TGF) or systemic hormones such as osteostatin, osteogenie and osteogrowth peptide (OGP), US 7,229,545 82 (Biomet Deutschland GrnbH) teaches bone-analogous coatings made of a collagen matrix mineralised with calcium phosphate, EP 1 442 755 A1 (Depuy Producls) a bioactive ceramic coaling comprising osteogenic proteins OP-1, BMP-7 and non-collagenous bone matrix proteins. Osteogenic activities have further been reported for fibroblast growth factor (FGF), transforming growth factor-ß (TGF-ß), platelet-derived growth factor (PDGF), insulin growlh factor (IGF) and family members of the foregoing. Zhang Q. et al describe in Biomacromolecules 2014, 15:84-94 the incorporation of recombinant human bone morphogenetic protein-2 (rhBMP-2) in an admixture with gelatin into a scaffold for a delivery of rhBMP-2 to stimulate improved bone regeneration via the supported growth and osteogenesis of human mesenchymal stem cells (hMSCs). EP 3231453 A1 (Immunodiagnostik AG) discloses a 3D-printed scaffold with an overall porosity of 20 to 90% having interconnecting pores, composed of bone cement and a resorbable polymer such as PLA. BSP is added to the body with interconnecting pores. The scaffold further comprises poly(Lys), poly(Gly-Pro-Hyp), tropocollagen or gelatin.

The prior art notwithstanding represents a problem as some implants often give rise to, for example, a condition called irnplantitis caused by an infection introduced during surgery. While irmplantitis can be dealt with by a course of antibiotics in the days prior and past surgery, a pre-emptive treatrment of peri-implantitis as well as improved wound and soft tissue healing would be more desirable. Aseptic loosening, inflammation reactions and long-term stability of endosseous implants further continue to remain a problem Despile bioactive coatings there is still a considerable period of time between surgery and osteointegration until when bone grafts and implants can withstand typical pressure, shear and tensile forces. Moreover, uncontrolled and undirectional growth of the callous also give rise to dysfunctional bone tissues.

The aforementioned bone replacement materials are usually offered as a pasty filling material or coated metallic implants. However, reconstruction surgery also requires dimensionally stable bone replacement bodies for the immediate repair of bonedefects as well as dimensionally stable bone replacement parts, which continue to harden after setting and which are absorbed in the course of healing. In this connection, the healing involves an ingrowth of bone tissue. So it is sought, a bone replacement body or a hardening bone substitute material that is absorbed and promote bone healing, bone remodeling and bone growth. The prior art represents the problem.

### SUMMARY OF THE INVENTION

The present invention and the scope thereof is defined by the appended claims. The more generic description of the invention is provided for illustrative purposes only. Embodiments referring to an implant scaffold or prosthesis composed of collagen, do not fall within the scope of the claimed subject-matter. Embodiments not falling under these claims are for reference purposes only. As a solution, the disclosure provides a polylactide prosthetic body for use in treating osseous defects and neogenesis of bone, obtained by the steps as defined in claim 1. polylactide scaffold is thereby obtained which induces tissue-directed ingrowth of bone tissue and repair and healing of damaged or diseased bone tissues and lesions. Further advantages and embodiments are described in the examples and the dependent claims.

In one aspect, the disclosure relates to a 3D-printed polylactide body for obtaining neogenesis of bone. The BSP may be recombinant human BSP.

In another aspect, the disclosure relates to a printed prosthesis for use in treating osseous defects and neogenesis of bone, wherein BSP is present in said mixture in a concentration from 1 µg BSP / mL to 100 µg/mL, preferably from 2 µg / rnL to 50 µg / mL, more preferably from 3 µg / rnL to 20 µg / mL.

The BSP may be contained as a BSP-collagen material wherein said admixed collagen solution comprises fibrillar collagen selected from the group of collagen Type I, II, III, V, or XI.

In another aspect, not falling within the scope of the claimed subject-matter, the disclosure relates to a material for treating osseous defects and neogenesis of bone, wherein said collagen solution comprises non-fibrillar collagen.

The disclosure also comprises, but does not fall within the scope of the claimed subject-matter, a prosthesis for treating osseous defects and neogenesis of bone wherein said collagen solution is a gel solution comprising hydrolyzed collagen.

In another aspect, the disclosure relates to a polylactide prosthesis further comprising a gellous material, wherein said BSP-collagen solution comprises from 0,1 % (w/v) to 50 % (w/v) collagen, preferably 0,5 % (w/v) to 20 % (w/v) collagen, more preferred from 1 % (w/v) to 10 % (w/v) collagen.

In one aspect, the disclosure relates to a polylactide prosthesis for use in treating osseous defects and neogenesis of bone which further comprises adsorbed natural or synthetic proteins which proteins are not originated from the complementary system.

In another aspect, the disclosure relates to a polylactide prosthesis for use in treating osseous defects and neogenesis of bone, which prosthesis comprises poly(Lys), poly(Glv-Pro-Hyp), tropocollagen and/or gelatine.

In one aspect, the disclosure relates to a polylactide prosthesis for use in treating osseous defects and neogenesis of bone, characterized in that its body has a total porosity from 10 to 90%, preferably from 20 to 70%, more preferred from 30 to 50%.

In another aspect, the disclosure relates to a use of the 3D-printed polylactide body as prosthesis for *in-vitro* neogenesis of bone in the reconstructive surgery, and to the 3D-printed polylactide body for use in in-vivo neogenesis in the reconstructive surgery.

In one aspect, not falling within the scope of the claimed subject-matter, the disclosure relates to a method of making a prosthesis having neo-osseogenic properties comprising the steps of providing a solution with a physiologically effective amount of human BSP, and mixing said solution with collagen .

The present disclosure offers the advantage that BSP incorporation in polylactide is easily feasible and results in a continuous protein release. BSP bio-functionalised polylactide bodies have a cell growth stimulating effect beneficial for tissue regeneration, while offering a safe, non-tumorigenic environment. At lower BSP concentrations, the gels enhance cell viability and sprout formation At higher BSP concentrations, osteoblast gene expression is enhanced. Notably, a concentration dependent effect can be observed in vivo already after three weeks from surgery.

The disclosure further provides a material for use in treating osseous defects and neogenesis of bone obtained by the steps of providing a material comprising polylactide, providing a physiologically effective amount of active human BSP, and combining said polylactide material and said physiologically effective amount of active human BSP before or during placement to obtain a material that furthers osseous repair and the healing of damage or diseased tissues and lesions. Advantages and embodiments of this disclosure are described in the examples and the dependent claims.

### BRIEF DESCRIPTION OF THE DRAWINGS

The present invention, its features and advantages will now be described by way of example only with reference to the accompanying drawings, wherein:

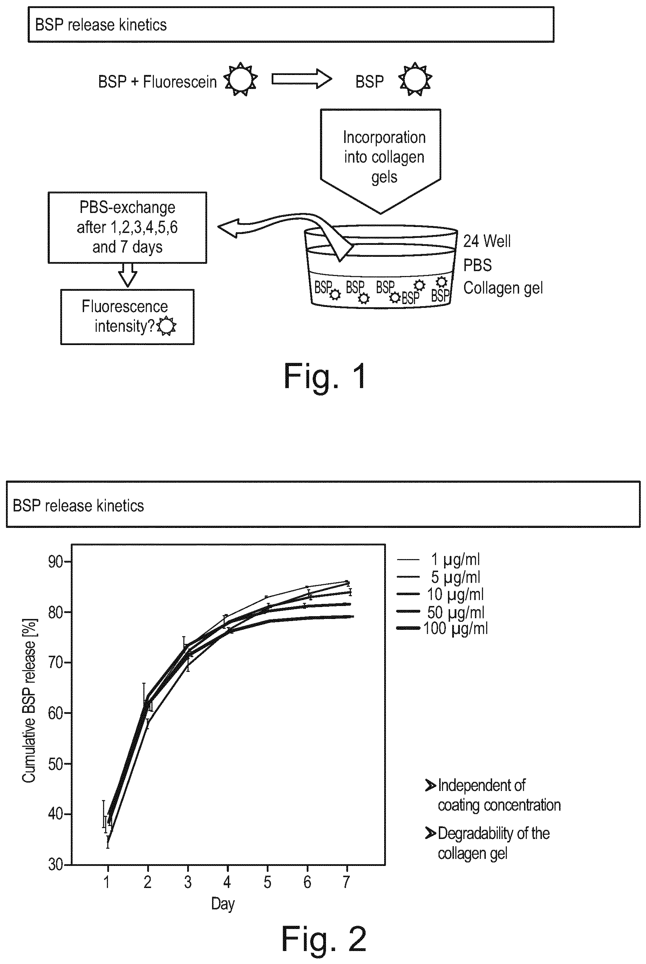

- Fig. 1: shows a schematic representation of a process for evaluating the properties of collagen gels (not falling within the scope of the claimed subject-matter) comprising a physiologically effective concentration of BSP;.
- Fig. 2: is a diagram showing BSP release kinetics in collagen gels (not falling within the scope of the claimed subject-matter);
- Fig. 3: is a schematic representation of the manufacturing steps of a 3-dimensional printed PLA replacement body which strings form pores of about 200 to 300 micrometer;
- Fig. 4: is a bar diagramm showing the release of BSP when coated with 1 or 10 µg BSP mixed with bovine collagen;
- Fig. 5: shows microscopic analyses of various cell lines when grown on a 3D-printed polylactide polymer modified by collagen, BSP and vitronectin;
- Fig. 6: shows microscopic analyses of osteosarcoma cells (SaOS-2) when grown on a 3D-printed polylactide body as described in Fig 3 comprising BSP or not;
- Fig. 7: shows microscopic analyses of endothelial cells (HUVEC) when grown on a 3D printed polylactide body as described in Fig. 3 comprising BSP or not;
- Fig. 8: shows microscopic analyses of a co-culture of osteosarcoma cells (SaOS-2) and endothelial cells (HUVEC) when grown on a 3D printed body containing BSP or not:
- Fig. 9: shows the steps of implanting a 3D-printed polylactide body for regeneration of a portion of the rat femur;
- Fig. 10: shows photos of crucial steps of the implant surgery and the removal of the implant after four and eight weeks of service;
- Fig. 11: are x-ray representations of implanted 3D-printed polylactide replacement bodies containing collagen and/or BMP-7 after surgery and 2, 4, 6, and 8 weeks of service;
- Fig. 12: are x-ray representations of implanted 3D-printed polylactide replacement bodies containing low and high concentrations of BSP after surgery and 2, 4, 6, and 8 weeks of service;
- Fig. 13: is schematic representation of the steps for evaluating *in vivo* the relevant parameters when using BSP collagen gels (not covered by the claims) in the calvarial bone defect model;
- Fig. 13A: displays images showing the bone regeneration in the rat calotte three weeks after surgery when using BSP collagen gels (not covered by the claims);
- Fig. 13B: displays images showing the bone regeneration in the rat calotte 8 weeks after surgery when using BSP collagen gels (not covered by the claims);
- Fig. 14: shows a bar diagram of the results of a quantitative examination of the effects of a BSP collagen gel (not covered by the claims) in the treatment of the calvarial bone defect model 3 weeks after surgery;
- Fig. 15A: shows hematoxylin-eosin stained histological sections of a treated bone defect using a BSP collagen gel (not covered by the claims) three weeks after surgery;
- Fig. 15B: shows hematoxylin-eosin stained histological sections of a treated bone defect using a BSP collagen (not covered by the claims) but eight weeks after surgery.

### DETAILED DESCRIPTION OF THE INVENTION

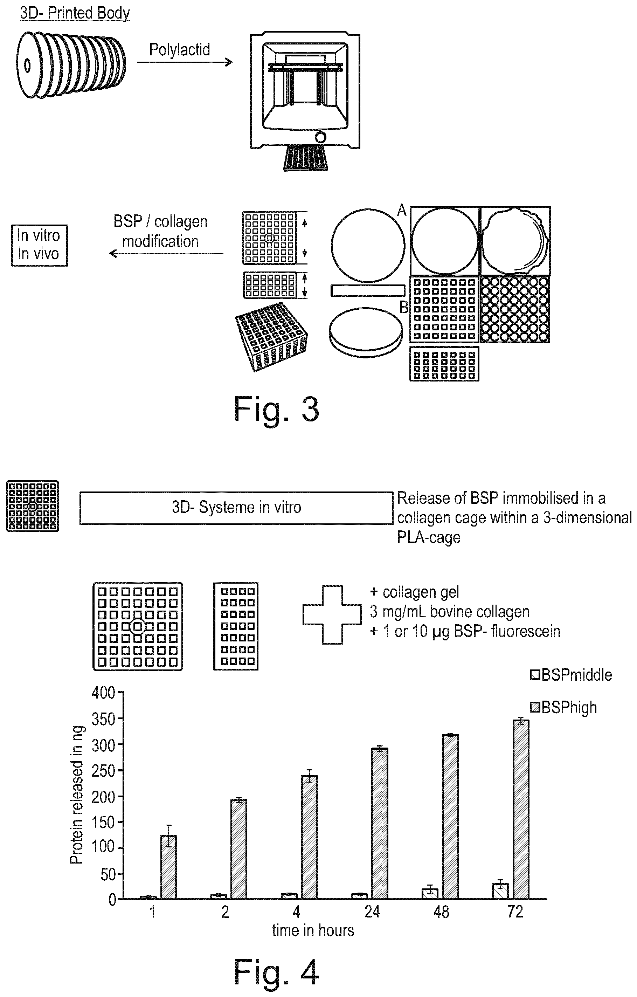

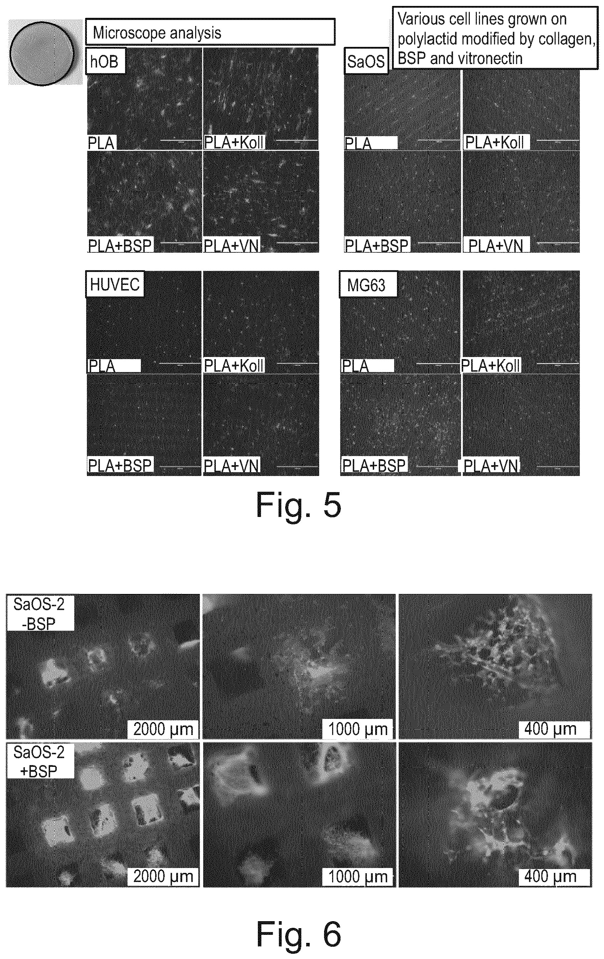

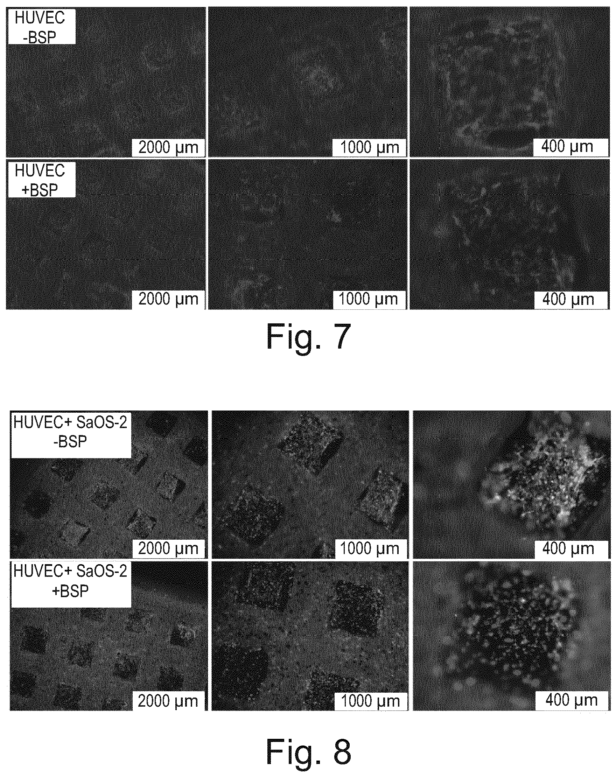

As a solution; the disclosure provides a prosthesis made of printed polylactide strings. The pores of the prosthesis have diameters of roughly 200 to 400 µm and therefore allow the entry of osteoblasts. The pores of the prosthesis may be filled and/or the strings be coated with a proteinaceous solution or gel comprising BSP and as a notable cofactor and stabilising agent collagen. The prosthesis for treating osseous defects and neogenesis of bone can be obtained by the steps shown in **Fig. 3**. The release of BSP and the degradability of the collagen gel (not covered by the claims) or polylactide was examined and controlled as exemplified in **Figs,** 1 and **2****.** Similar can be expected for degradable polylactide strings. The pores of the printed prosthesis may be filled either with a solution or a gel comprising BSP and collagen as exemplified in **Fig.** 4. In this way a physiological degradable body is provided as both the polylactide strings of the printed body as well as the collagen gel are degradable or absorbed in the course of the healing process. **Figs. 5-8** show various cell lines, notably human osteoblasts (hOB) and human osteosarcoma cells (SaOS-2), as well as HUVECs (human primary umbilical vein endothelial cells) grown in the presence and absence of BSP, vitronectin, collagen, PLA as indicated for comparative purposes. Generally, the presence of BSP lead to increased growth and differentiation of cells and notably hOBs.

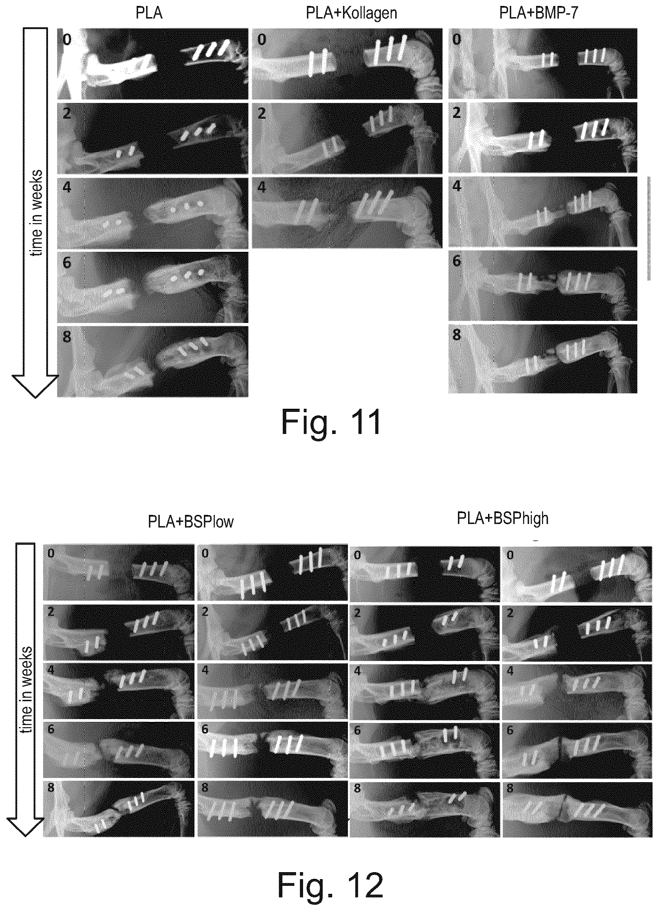

The enormous advantage compared to the state-of-the-art employing bone morphogenic proteins (BMPs) is that BSP does not induce overgrowing of bone tissue and that the described combination of a printed polylactide body furthers directed or conducted neogenesis of bone material as exemplified in Figure 12. Figure 11 shows the 3D printed prosthesis but coated or trenched with collagen or bone morphogenic protein 7 (BMP-7). The bone morphogenetic proteins (BMPs) are growth factors and received their name by the discovery that they can induce the formation of bone and cartilage. BMPs are now considered morphogenetic signals which orchestrate tissue architecture throughout the body. The functioning of BMP signals in physiology is further emphasized by the multitude of roles for dysregulated BMP signalling in pathological processes. Recombinant human BMPs (rhBMPs) are meanwhile used in orthopedic applications and recombinant human BMP-2 and BMP-7 have received Food and Drug Administration (FDA)-approval for some uses. However, rhBMP-2 and rhBMP-7 can cause an overgrowing of bone.

While new additional bone tissue is formed by the signal action of BMP-7, the bone tissue is not formed in physiological or natural direction of the femur or calvarial defect. In other words, the newly formed bone tissue is not directional but callous-like. Even in the case of even a high concentration of BSP (see Fig 12, eight weeks), we found that a prosthesis containing BSP - adsorbed or contained within a collagen gel (not covered by the claims) or polylactide - does not only induce and support the formation of new bone tissue but that the BSP induces in this combination a tissue-directed formation of bone. It is therefore further obvious to print and place a 3D-printed body or prosthesis in a way that the pores are osseous conductive so that the cell and osseous-inductive signaling of the BSP can be fully used.

### Example 1 - BSP coated printed prosthesis in rat femur

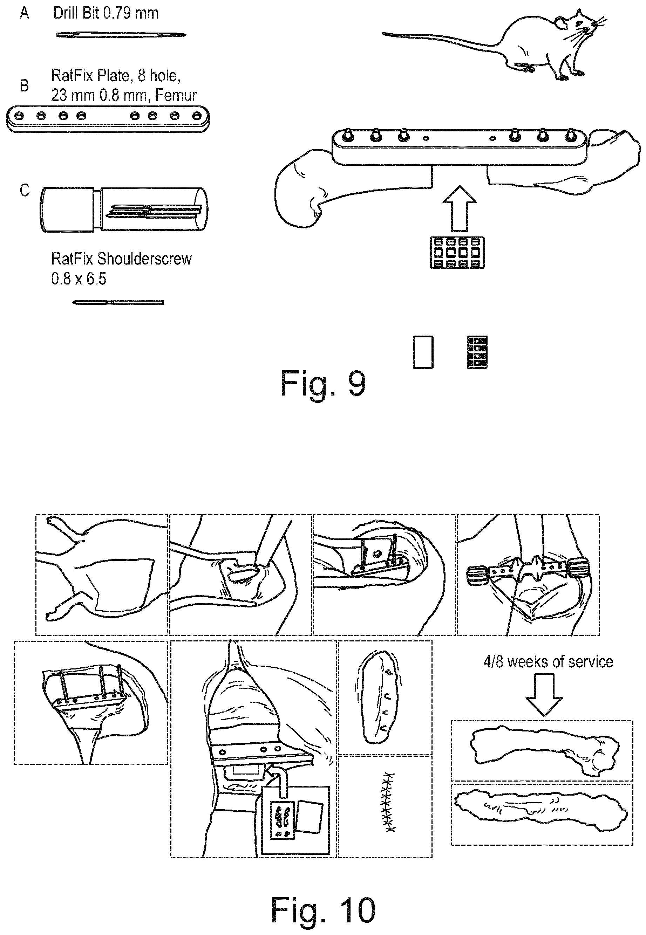

The animal experiment was approved by the competent Rhineland-Palatinate State Investigation Office (LUA). The steps of this animal experiment can be taken from Figs. 9 and 10. In brief, a piece of the rat femur was cut out as indicated and repaired by a 3-D printed polylactide prosthesis which was put in place as indicated using an 8-hole fixing plate. Fig. 9 shows the principle and Fig 10 the surgery steps. The rat femur was X-rayed immediately after surgery and after 2, 4, 6 and 8 weeks as shown in Figs. 11 and 12. The fixing plate and the prosthesis are not sufficiently electron dense to be visible. The femurs were further histologically examined (results not shown).

The enormous advantage compared to the state-of-the-art (Fig. 11) employing bone morphogenic proteins (BMPs) or collagen is that BSP does not induce overgrowing of bone tissue and that the described combination of a printed polylactide body furthers directed or conducted neogenesis of bone material as exemplified in Figure 12 (see in particular X-ray photos after eight weeks). Figure 11 shows printed polylactide prosthesis coated or trenched with collagen or bone morphogenic protein 7 (BMP-7). The bone morphogenetic proteins (BMPs) induced formation of bone but the growth of bone was not directional. BMPs are generally considered morphogenetic signals which orchestrate tissue architecture throughout the body. The coated BMP however leads to dysregulated BMP signalling in pathological processes while recombinant human BMP-2 and BMP-7 have received Food and Drug Administration (FDA)-approval for some orthopedic applications. However, rhBMP-2 and rhBMP-7 cause overgrowth and non-directional growth of bone. As shown in Fig. 11, while additional bone tissue is formed by the action of BMP-7, the additional bone tissue is not formed in the direction of the femur. Consequently, the newly formed bone tissue is non-directional but callous-like and therefore not serving or assisting the functions of the femur.

Referring to Fig. 12 (eight weeks), we could observe that a placed printed polylactide prosthesis with low or high concentration of BSP induced directional formation of new bone tissue. The prosthesis was a porous body containing BSP, adsorbed onto the polylactide strings. It is therefore obvious to print and place the prosthesis in a way that the pores are osseous conductive so that the cell and osseous-inductive signaling of the BSP is fully effective.

### Reference

### Example 2 - BSP collagen prosthesis in treating in a calvarial bone defect

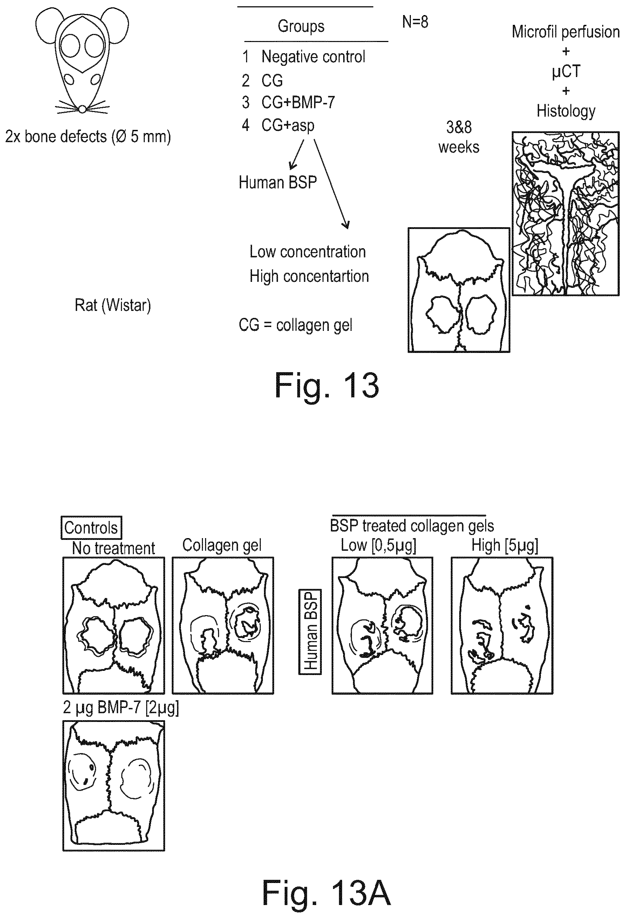

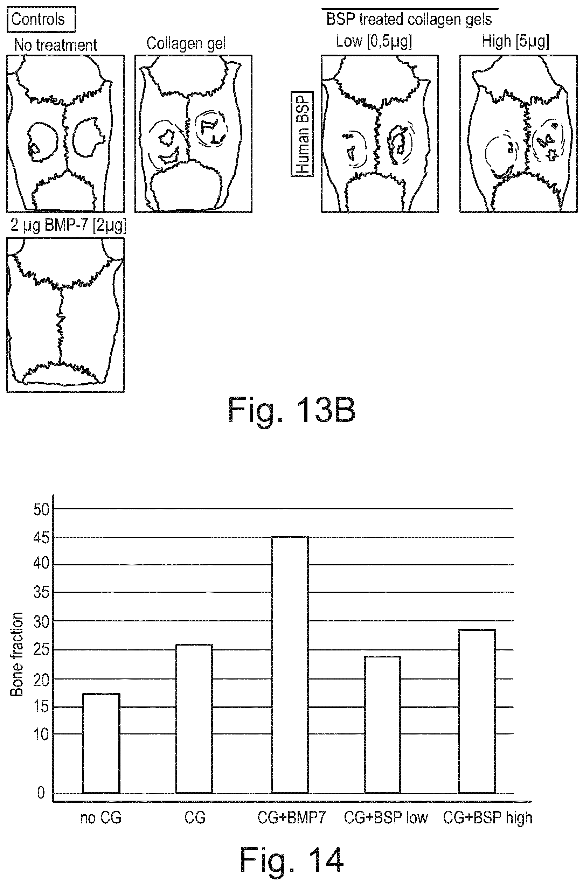

Referring to Fig. 13, the prosthesis was used to treat a calvarial bone defect. The animal experiment was approved by the competent Rhineland-Palatinate State Investigation Office (LUA). The rats were operated under general anesthesia and placed in each case two boreholes with a diameter of 2.5 mm - a bone defect of critical size. Then, BSP collagen gels were placed into the borehole to cover it up. After an observation periods of 3 and 8 weeks, the animals were anesthetized and decapitated.

The skulls were fixed in formalin for one week and prepared for IJeT imaging. The animal experiment included following groups:
a) Negative control: i) untreated borehole defect, and ii) treated borehole defect with collagen only.
b) Positive control: treated borehole defect with BMP-7 (2 µg).
c) Experiment: i) BSP treated collagen gels with 0,5 µg , and ii) BSP treated collagen gels with 5 µg .

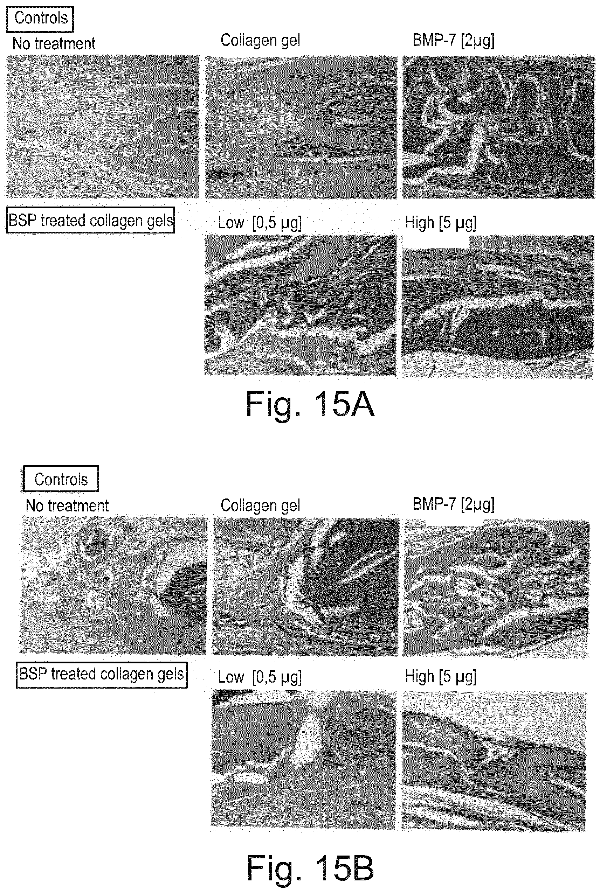

The BSP treated animals showed improved osseous regeneration compared to controls already three weeks after surgery. Notably, the growth of bone tissue was more homogenous and functional in the BSP treated animals, cf **Figs. 15A-B.** The additional bone growth in the positive control (BMP-7) was similar but with signs of osseous overgrowth.

### CONCLUSIONS

A prosthetic polylactide scaffold material for use in treating osseous defects and neogenesis of bone, obtained by the steps of printing a scaffold composed of strings of polylactide and porous microstructures which allow passage and ingrowth of bone tissue. A soluble mixture of BSP is provided, and BSP is applied onto the strings and in the pores of the printed body to obtain a prosthetic material which induces tissue-directed ingrowth of bone tissue as well as repair and healing of damaged or diseased bone tissues and lesions. The prosthetic material has thereby been made osseo-inductive and osseo-conductive. The BSP in the prosthetic scaffold material induces a tissue-directed growth of osseous tissue. No undirectional callous or overgrowing bone and cartilage tissue is observed.

## Claims

1. A 3D-printed polylactide body as an implant scaffold or prosthesis wherein the polylactide body is composed of biodegradable polylactide (PLA) and forms cage-like microstructures, pores and channels having a size of 100 to 1000 micrometres size to allow passage and ingrowth of osteoblasts and osteoclasts, obtained by the steps of:-
providing a gel-like or liquid solution containing biodegradable polylactide,
providing a physiologically effective amount of active human BSP, and
combining said gel-like or liquid polylactide material with said physiologically effective amount of active BSP and/or
treating and/or impregnating said implant scaffold or prosthesis with said mixture prior to surgical placement to obtain a 3D-printed implant scaffold or prosthesis that effectively releases BSP and promotes tissue-directed repair and healing of damaged or diseased tissues and lesions.

2. The 3D-printed scaffold or prosthesis of claim 1 wherein BSP is human recombinant BSP.

3. The 3D-printed scaffold or prosthesis of claim 1 or 2, wherein BSP is present in a concentration from 1 µg/mL to 100 µg/mL, preferably from 2 µg/mL to 50 µg/mL, more preferably from 3 µg/mL to 20 µg/mL.

4. The 3D-printed scaffold or prosthesis of any claim 1 to 3, comprising a collagen solution of fibrillar collagen selected from the group of collagen Type I, II, III, V, or XI.

5. The 3D-printed scaffold or prosthesis of any claim 1 to 3, comprising a collagen solution of non-fibrillar collagen.

6. The 3D-printed scaffold or prosthesis of any claim 1 to 5, wherein said collagen is a gel solution comprising hydrolysed collagen.

7. The 3D-printed scaffold of any claim 1 to 6, wherein said gel solution comprises from 0,1 % (w/v) to 50 % (w/v) collagen, preferably 0,5 % (w/v) to 20 % (w/v) collagen, more preferred from 1 % (w/v) to 10 % (w/v) collagen.

8. The 3D-printed scaffold or prosthesis of any claim 1 to 7, further comprising poly(Lys), poly(Gly-Pro-Hyp), tropocollagen or gelatine.

9. The 3D-printed scaffold or prosthesis of any claim 1 to 8, having a total porosity from 10 to 90%, preferably from 20 to 70%, more preferred from 30 to 50%.

10. The 3D-printed scaffold or prosthesis of any claim 1 to 9 for use in *in-vivo* neogenesis of a homologous bone implant.

11. Use of the 3D-printed scaffold or prosthesis of any claim 1 to 9 for *in-vitro* neogenesis of a homologous bone implant.

## Patentansprüche

1. 3D-gedruckter Polylactidkörper als Implantatgerüst oder -prothese, wobei der Polylactidkörper aus biologisch abbaubarem Polylactid (PLA) besteht und käfigartige Mikrostrukturen, Poren und Kanäle mit einer Größe von 100 bis 1000 Mikrometern bildet, die den Durchgang und das Einwachsen von Osteoblasten und Osteoklasten erlauben, erhalten durch die Schritte
Bereitstellung einer gelartigen oder flüssigen Lösung, die biologisch abbaubares Polylactid enthält,
Bereitstellung einer physiologisch wirksamen Menge aktives humanes BSP, und Vereinigen des gelartigen oder flüssigen Polylactidmaterials mit der physiologisch wirksamen Menge aktiven BSP und/oder
Behandeln und/oder Imprägnieren des Implantatgerüsts oder der Prothese mit dem Gemisch vor seiner chirurgischen Platzierung, so dass man ein 3D-gedrucktes Implantatgerüst oder eine Prothese erhält, die wirksam BSP freisetzen und eine gewebeentsprechende Reparatur und Heilung von beschädigtem oder krankem Gewebe und Läsionen fördert.

2. 3D-gedrucktes Gerüst oder die Prothese nach Anspruch 1, wobei das BSP humanes rekombinantes BSP ist.

3. 3D-gedrucktes Gerüst oder die Prothese nach Anspruch 1 oder 2, wobei BSP in einer Konzentration von 1 µg/mL bis 100 µg/mL, vorzugsweise von 2 µg/mL bis 50 µg/mL, noch bevorzugter von 3 µg/mL bis 20 15 µg/mL vorhanden ist.

4. 3D-gedrucktes Gerüst oder die Prothese nach einem der Ansprüche 1 bis 3, enthaltend eine Kollagenlösung aus fibrillärem Kollagen, ausgewählt aus der Gruppe von Kollagen Typ I, II, III, V oder XI.

5. 3D-gedrucktes Gerüst oder die Prothese nach einem der Ansprüche 1 bis 3, das eine Kollagenlösung aus nicht-fibrillärem Kollagen enthält.

6. 3D-gedrucktes Gerüst oder die Prothese nach einem der Ansprüche 1 bis 5, wobei das Kollagen eine Gellösung ist, die hydrolysiertes Kollagen umfasst.

7. 3D-gedrucktes Gerüst nach einem der Ansprüche 1 bis 6, wobei die Gellösung 0,1 % (Gew./Vol.) bis 50 % (Gew./Vol.) Kollagen, vorzugsweise 0,5 % (Gew./Vol.) bis 20 % (Gew./Vol.) Kollagen, noch bevorzugter 1 % (Gew./Vol.) bis 10 % (Gew./Vol.) Kollagen enthält.

8. 3D-gedrucktes Gerüst oder die Prothese nach einem der Ansprüche 1 bis 7, enthaltend zudem Poly(Lys), Poly(Gly-Pro-Hyp), Tropokollagen oder Gelatine.

9. 3D-gedrucktes Gerüst oder die Prothese nach einem der Ansprüche 1 bis 8, das eine Gesamtporosität von 10 bis 90 % hat, vorzugsweise von 20 bis 70 %, noch bevorzugter von 30 bis 50 %.

10. 3D-gedrucktes Gerüst oder die Prothese nach einem der Ansprüche 1 bis 9 zur Verwendung bei der In-vivo-Neogenese eines homologen Knochenimplantats.

11. Verwendung des 3D-gedruckten Gerüsts oder der Prothese nach einem der Ansprüche 1 bis 9 zur In-vitro-Neogenese eines homologen Knochenimplantats.

## Revendications

1. Corps en polylactide imprimé en 3D servant de support d'implant ou de prothèse, dans lequel le polylactide est composé de polylactide biodégradable (PLA) et forme des microstructures en forme de cage, des pores et des canaux d'une taille de 100 à 1 000 micromètres pour permettre le passage et la croissance des ostéoblastes et des ostéoclastes, le corps en polylactide étant obtenu par les étapes de :
- fournir une solution gélifiée ou liquide contenant du polylactide biodégradable ;
- fournir une quantité physiologiquement efficace de BSP humaine active.
- combiner le matériau polylactide gélifié ou liquide avec ladite quantité physiologiquement efficace de BSP active, et/ou
- traiter et/ou imprégner ledit échafaudage d'implant ou ladite prothèse avec ledit mélange avant la mise en place chirurgicale afin d'obtenir un échafaudage d'implant ou une prothèse imprimée en 3D qui libère efficacement la BSP et favorise la réparation et la cicatrisation des tissus et des lésions endommagés ou malades.

2. L'échafaudage ou la prothèse imprimé(e) en 3D de la revendication 1, dans lequel la BSP est une BSP recombinante humaine.

3. L'échafaudage ou la prothèse imprimé(e) en 3D de la revendication 1 ou 2, dans lequel la concentration de BSP est de 1 µg/mL à 100 µg/mL, de préférence de 2 µg/mL à 50 µg/mL, plus préférentiellement de 3 µg/mL à 20 µg/mL.

4. L'échafaudage ou la prothèse imprimé(e) en 3D des revendications 1 à 3, comprenant une solution de collagène fibrillaire choisie dans le groupe des collagènes de type I, II, III, V ou XI.

5. L'échafaudage ou la prothèse imprimé(e) en 3D des revendications 1 à 3, comprenant une solution de collagène non fibrillaire.

6. L'échafaudage ou la prothèse imprimé(e) en 3D de l'une des revendications 1 à 5, dans lequel le collagène est sous forme de gel comprenant du collagène hydrolysé.

7. L'échafaudage imprimé en 3D selon l'une des revendications 1 à 6, dans lequel ladite solution de gel comprend de 0,1 % (p/v) à 50 % (p/v) de collagène, de préférence de 0,5 % (p/v) à 20 % (p/v) de collagène, plus préférentiellement de 1 % (p/v) à 10 % (p/v) de collagène.

8. L'échafaudage ou la prothèse imprimé(e) en 3D de l'une des revendications 1 à 7, comprenant en outre du poly(Lys), du poly(Gly-Pro-Hyp), du tropocollagène ou de la gélatine.

9. L'échafaudage ou la prothèse imprimé(e) en 3D selon l'une des revendications 1 à 8 présente une porosité totale comprise entre 10 et 90 %, de préférence entre 20 et 70 %, plus préférentiellement entre 30 et 50 %.

10. L'échafaudage ou la prothèse imprimé(e) en 3D selon l'une des revendications 1 à 9 est destiné à la néogenèse in vivo d'un implant osseux homologue.

11. Utilisation de l'échafaudage ou de la prothèse imprimés en 3D selon l'une des revendications 1 à 9 pour la néogenèse in vitro d'un implant osseux homologue.
